# EUROPEAN PATENT APPLICATION

(11) **EP 3 692 932 A1**
(43) Date of publication of application: **12.08.2020**
(21) Application number: 20155929.1
(22) Date of filing: 06.02.2020
(51) Int. Cl.: A61B 17/16, A61F 2/46

(54) **MULTIFUNCTION DEVICE**

(30) Priority: 07.02.2019 IT 201900001801; 24.09.2019 IT 201900017084
(71) Applicant: HPF S.R.L., 33034 Fagagna (IT)
(72) Inventor: Lualdi, Gabriele, 33034 Fagagna (IT)
(74) Representative: Petraz, Gilberto Luigi

(57) **Abstract**

Multifunction device for prosthetic surgery able to be converted from a milling configuration, to be used as a milling device (10a) for hip prosthetic surgery, to a positioning configuration, to be used as a positioning device (10b) to position an acetabular cup (11) of a hip prosthesis.

## Description

### FIELD OF THE INVENTION

The present invention concerns a multifunction device for hip prosthetic surgery interventions.

The multifunction device is of the convertible type, in that it can be used as a milling device, for example to make an acetabular seating to install an acetabular cup of a hip, or as a positioning device, for example to position and release the acetabular cup of a hip in the acetabular seating previously made.

### BACKGROUND OF THE INVENTION

The devices that can be used during hip prosthetic surgery interventions are known.

In particular, it is known that during the steps of preparing the acetabular seating and implanting the corresponding prosthesis, many and different devices are required, the choice and conformation of which can also depend on the chosen surgical access route such as, for example, the posterolateral route or the anterior route.

In normal operating practice milling devices are used, to make coordinated and mating acetabular seatings suitable for the disposition and implant of corresponding acetabular cups, and positioning devices to allow the correct positioning, also angular, of the prostheses as above in the acetabular seatings and for their release into position.

The surgeon is therefore, on each occasion, called upon to choose in advance the devices to be used during the surgical operation, based on the chosen access route and, moreover, to use different devices for the above-described operations of milling the acetabular seating, positioning and release of the prosthesis.

In particular, known milling devices comprise a handling body provided with a proximal end, to which a drive member of the manual or motorized type is operatively associated, and a distal end, to which is operatively associated a milling tool that can be used for the realization of hemispherical acetabular seatings, or in any case with a spherical cap, suitable to install coordinated acetabular cups of the hip prostheses.

The rotational motion supplied by the drive member is transmitted to the milling tool through a transmission unit inside the handling body itself, for example a transmission system with cardan joints.

However, both the drive member and also the milling tool have connection portions able to be associated only with the determinate handling body which has attachment portions, mating with the connection portions as above, different from each other.

This requires the surgeon to purchase an entire tool kit from a single supplier.

Often, however, there is the need to choose a handling body that has a specific conformation, a milling tool or a drive member from different manufacturing companies, for example for practical reasons, but also due to needs related to the characteristics of the patient to be operated on such as the underlying pathology, age, body weight or the suffering of possible allergies to some materials.

In this regard, known milling devices are typically made of steel. The latter is an allergic material that, due to wear, rubbing or impacts, can release traces of metals, such as nickel and chromium, which can induce allergic reactions in the patient undergoing surgical treatment.

The positioning devices also comprise a handling body which at the proximal end, or in a nearby position, has an ergonomic grip, or handle, to allow the surgeon to correctly position the acetabular cup in the acetabular seating made, and at the distal end has a positioning element to which the acetabular cup as above can be associated, temporarily.

The desired angular position of the acetabular cup can be reached by acting on a transmission member that allows the rotation of the positioning element and therefore of the acetabular cup.

The transmission member is driven manually and can be provided in correspondence with the proximal end or in another position along the handling body, and is connected to the positioning element by means of a suitable transmission unit.

Once correctly positioned, the acetabular cup is released into the acetabular seating by means of a release mechanism, for example a pressure or spring mechanism, activated in correspondence with the transmission member by means of a trigger, or by means of a lever or by mechanical impact.

However, in the event the release mechanism is activated by mechanical impact, the stresses produced are discharged on the transmission unit causing its rapid deterioration.

The handling body of the positioning devices typically has an asymmetrical curved conformation to allow the surgeon to easily reach the acetabular seating.

One disadvantage of this conformation is that the mechanical impact to activate the release mechanism causes a moment of forces which tends to misalign, both axially and also angularly, the positioned acetabular prosthesis.

It is also known that the devices described above have to be subjected to washing and sterilization operations after each intervention, and have to therefore be made of a material suitable to withstand the attack of aggressive chemical agents, such as iodine-based disinfectants, and to withstand temperatures in the order of about 130°C.

In addition, known devices are complicated and consist of numerous components, which are difficult to disassemble, for sterilization, and to assemble, in order to be available during the surgical operation.

It is not unusual, in fact, that an incorrect assembly of the devices as above can cause malfunctions during the surgical operation.

There is therefore the need to perfect a device for prosthetic surgery that can overcome at least one of the disadvantages of the state of the art.

In particular, one purpose of the present invention is to provide a multifunction device for prosthetic surgery which can selectively be a milling device or a positioning device.

Another purpose of the present invention is to provide a multifunction device for prosthetic surgery with which it is possible to operatively associate any drive member, any milling tool, and any positioning element whatsoever with respect to both the proximal and also the distal end.

Another purpose of the present invention is to provide a multifunction device for prosthetic surgery provided with a release mechanism of the acetabular cup the activation of which does not cause a deterioration of the transmission unit.

Another purpose of the present invention is to provide a multifunction device for prosthetic surgery made of hypoallergenic and biocompatible material.

Another purpose of the present invention is to provide a multifunction device for prosthetic surgery made up of a limited number of components.

Another purpose of the present invention is to provide a multifunction device for prosthetic surgery which is easy to disassemble and assemble.

The Applicant has studied, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

In accordance with the above purposes, a multifunction device for prosthetic surgery able to be converted from a milling configuration, to be used as a milling device for hip prosthetic surgery, to a positioning configuration, to be used as a positioning device to position an acetabular cup of a hip prosthesis comprises:
- a single tubular oblong handling body 12, which develops along an operating axis, provided with a distal end and a proximal end opposite each other, inside the handling body there is a unit to transmit the rotary motion from the distal end to the proximal end, the transmission unit ending in respective attachment portions, respectively distal and proximal, which are interchangeable with each other;
- one or more acetabular milling cutters able to be releasably connected to one of the attachment portions in the milling configuration as above;
- a positioning element able to be releasably fastened on one side directly to one of the attachment portions and on the other side to an acetabular cup of a hip prosthesis, in the positioning configuration as above;
- a transmission element able to be releasably fastened to one of the attachment portions opposite the attachment portion to which the acetabular milling cutter or the positioning element is fastened, the transmission element being configured so that, in the milling configuration or respectively in the positioning configuration as above, it can be used to transmit, by means of the transmission unit, a rotation from one of the attachment portions to the other of the attachment portions, respectively associated with a specific acetabular milling cutter or with an acetabular cup associated with the positioning element, or again the transmission element, in the positioning configuration as above, is able to be struck by a striker member in order to transmit an impact force, through the handling body and the positioning element, to forcefully position the acetabular cup associated with the positioning element.

In accordance with some embodiments, there is provided a method to use the multifunction device for prosthetic surgery in a milling configuration, to be used as a milling device for hip prosthetic surgery, and in a positioning configuration, to be used as a positioning device to position an acetabular cup of a hip prosthesis.

### BRIEF DESCRIPTION OF THE DRAWINGS

These and other aspects, characteristics and advantages of the present invention will become apparent from the following description of some embodiments, given as a non-restrictive example with reference to the attached drawings wherein:
- fig. 1 is a lateral view of a handling body of a multifunction device for multifunction prosthetic surgery in accordance with some embodiments;
- fig. 2 is an exploded view of fig. 1;
- fig. 3 is an exploded lateral view of fig. 1;
- fig. 4 is a perspective view of a component of fig. 2;
- fig. 5 is a section along a vertical plane of fig. 4;
- fig. 6 is a perspective view of the multifunction device for prosthetic surgery, in accordance with the embodiment of fig. 1, when it is used as a milling device;
- fig. 7 is an exploded view of fig. 6;
- fig. 8 is a section view of two components of fig. 7;
- fig. 9 is a section view of two components of fig. 7;
- fig. 10 is a perspective view of the multifunction device for prosthetic surgery, in accordance with the present invention, when it is used as a positioning device;
- fig. 11 is an exploded view of fig. 10;
- fig. 12 is a perspective view of a detail of fig. 11;
- fig. 13 is a section view of two components of fig. 11;
- fig. 14 is a section view of three components of fig. 11;
- fig. 15 is a lateral view of a handling body of a multifunction device for multifunction prosthetic surgery according to another embodiment;
- fig. 16 is an exploded view of fig. 15;
- fig. 17 is an exploded lateral view of fig. 15;
- fig. 18 is a perspective view of a component of fig. 16;
- fig. 19 is a lateral view of fig. 18;
- fig. 20 is a section along a vertical plane of fig. 18;
- fig. 21 is a perspective view of the multifunction device for prosthetic surgery, in accordance with the embodiment of fig. 15, when it is used as a milling device;
- fig. 22 is an exploded view of fig. 21;
- fig. 23 is a section view of two components of fig. 21;
- fig. 24 is a section view of two components of fig. 21;
- fig. 25 is a perspective view of the multifunction device for prosthetic surgery, in accordance with the embodiment of fig. 15, when it is used as a positioning device;
- fig. 26 is an exploded view of fig. 25;
- fig. 27 is a perspective view of a detail of fig. 11;
- fig. 28 is a view of an enlarged detail of fig. 25;
- fig. 29 is a section view of three components of fig. 28;
- fig. 30 is a view of an enlarged detail of fig. 25;
- fig. 31 is a section view of three components of fig. 30.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We will now refer in detail to the various embodiments of the invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments to produce another embodiment. It is understood that the present invention shall include all such modifications and variants.

Embodiments described using the attached drawings concern a multifunction device for prosthetic surgery indicated as a whole with reference number 10a and 10b in the attached drawings, according to the condition in which it is converted and used.

The multifunction device is able to be converted to be used, in accordance with a milling configuration, as a milling device 10a for hip prosthetic surgery, in particular to make an acetabular seating, and, in accordance with a positioning configuration, as a positioning device 10b for positioning an acetabular cup 11 of a hip prosthesis in the acetabular seating as above.

The multifunction device comprises a single tubular oblong handling body 12 which develops along an operating axis A.

The single handling body 12 is provided with a distal end 13 and with a proximal end 14 opposite each other.

Inside the handling body 12 there is a transmission unit 27 of the rotary motion from the distal end 13 to the proximal end 14, the transmission unit 27 ending in respective attachment portions 15, 16 which are interchangeable with each other.

Hereafter, for ease of explanation, reference may also be made to a distal attachment portion 15 and a proximal attachment portion 16, meaning, however, that they can be used indifferently for the functions that will be described in the following document, since they are interchangeable.

The multifunction device, also, comprises one or more acetabular milling cutters 17 able to be releasably connected to one of the attachment portions 15, 16 in the milling configuration as above.

Furthermore, the multifunction device comprises a positioning element 20 able to be releasably fastened on one side directly to one of the attachment portions 15, 16 and on the other side to an acetabular cup 11 of a hip prosthesis, in said positioning configuration.

Furthermore, the multifunction device comprises a transmission element 21 able to be releasably fastened to one of the attachment portions 16, 15 opposite the attachment portion 15, 16 to which the acetabular milling cutter 17 or the positioning element 20 is fastened, the transmission element 21 being configured so that, in the milling configuration as above or respectively in the positioning configuration as above, it can be used to transmit, by means of the transmission unit 27, a rotation from one of the attachment portions 16, 15 to the other of the attachment portions 15, 16, respectively associated with the specific acetabular milling cutter 17 or with the acetabular cup 11 associated with the positioning element 20.

Or, again, the positioning element 20, in the positioning configuration, is able to be struck by a striker member to transmit an impact force, through the handling body 12 and the positioning element 20, to forcefully position the acetabular cup 11 associated with the positioning element 20.

In accordance with some embodiments, the handling body 12 has an elongated conformation in the direction of the operating axis A as above passing through the attachment portions 15, 16 and comprises a first shell 22 and a mating second shell 23 able to be stably coupled to each other in a releasable manner in order to house, inside, the transmission unit 27 as above.

The transmission unit 27 allows the transmission of the rotary motion, generated by the drive member, from the proximal portion 16 to the distal portion 15 or vice versa.

Advantageously the first shell 22 and the second shell 23 are identical and specular and are coupled to each other by means of tightening ring nuts 24 present on both the distal end 13 and also the proximal end 14, coaxially with the respective attachment portions 15, 16.

The configuration described, allows to simplify the assembly and disassembly of the handling body 12, both for the surgical operation and also to sterilize all its components.

The handling body 12 has at least one inclined segment to aid the surgeon in moving and positioning the multifunction device toward the patient's acetabulum during the surgical operation.

Advantageously, the handling body 12 has two specular inclined segments 12a, 12c, in correspondence with the distal attachment portion 15 and the proximal attachment portion 16 angled with respect to said operating axis A, and a linear segment 12b, comprised between the inclined segments 12a, 12c, parallel to the operating axis A.

Advantageously, the inclined segments 12a, 12c are angled with respect to the operating axis A by the same angle α comprised between about 15° and about 35°.

The handling body 12 has a conformation symmetrical with respect to a central axis S orthogonal to the operating axis A and passing through the center of the handling body 12.

The handling body 12 has a conformation symmetrical with respect to a coupling plane passing through the operating axis A and with respect to which the first shell 22 and the second shell 23 are coupled.

This conformation of the handling body 12 is important both when the multifunction device is used as a milling device 10a to make an acetabular seating, and also when the device is used as a positioning device 10b to position and release the acetabular cup 11.

Furthermore, the symmetrical conformation of the handling body 12 allows it to be used indiscriminately in one direction or another of the operating axis A as above.

The transmission unit 27 comprises rotation shafts 28a, 28b, 28c, respectively corresponding to the segments 12a, 12b, 12c, and connected by cardan joints 29 which allow the transmission of the rotary motion with respect to the incident directions of the segments 12a, 12b, 12c.

In this specific case, the rotation shafts 28a, 28c are the two outermost ones and are respectively connected on one side to the rotation shaft 28b and on the other, always by means of a cardan joint 29, to a connection interface 30 configured to be associated respectively with the proximal attachment portion 16 and with the distal attachment portion 15.

The transmission unit 27 is operatively inserted inside the handling body 12, between the first shell 22 and the second shell 23.

For this purpose, the first shell 22 and the second shell 23 comprise a plurality of support cavities 25 and passage cavities 26 suitable to contain, once coupled, the transmission unit 27.

In particular, the transmission unit 27 comprises bearings 31 which, during use, are positioned resting in the support cavities 25 and allow the rotation of the shafts 28a, 28b, 28c without any friction against the internal walls of the first shell 22 and the second shell 23.

Especially when the multifunction device is used as a milling device 10a, the bearings 31, as well as preventing friction of the rotation shafts 28a, 28b, 28c, are configured to absorb the vibrations that are generated due to the inertia of the rotating members allowing a greater control of the milling device 10a by the surgeon.

According to some embodiments, the attachment portions 15, 16 are, in particular, the same and interchangeable each comprising a base 32, a coupling head 35 and a plate 34 located intermediate.

The base 32 is provided with a connection compartment 33 with a shape mating with that of the connection interface 30 for the operative connection to the transmission unit 27.

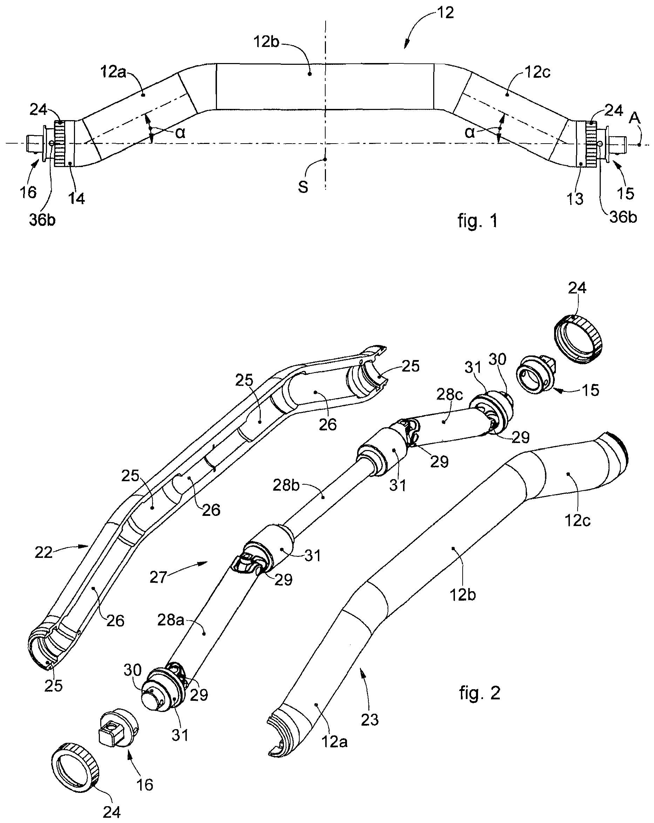

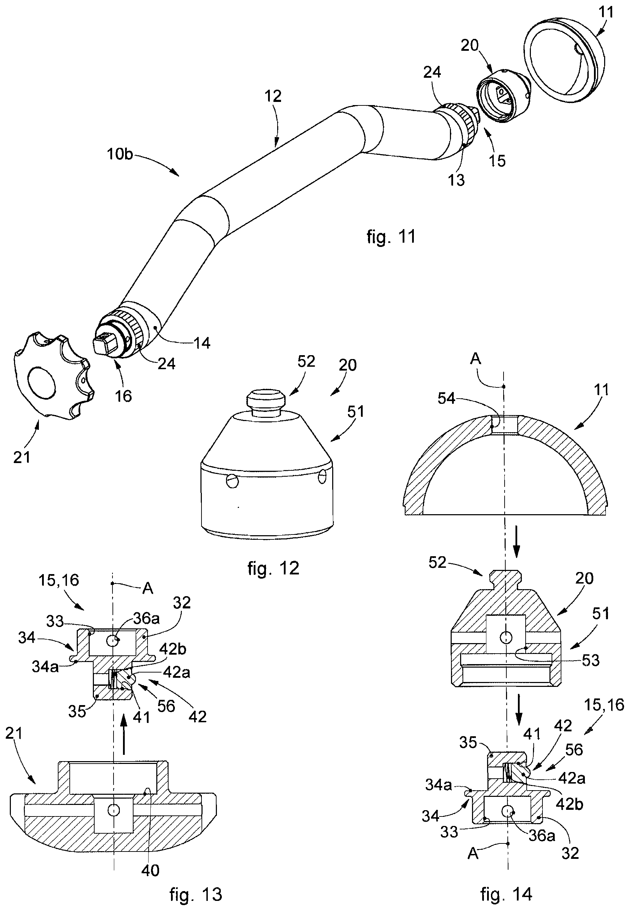

In accordance with some embodiments, shown in figs. 1-14, both the base 32 and also the connection interface 30 are provided, on a lateral surface, respectively with connection holes 36a, 36b which during use are aligned in order to house an interference element which allows to make respectively the distal attachment portion 15 and the proximal attachment portion 16 integral at least temporarily with the transmission unit 27.

In accordance with possible solutions, the interference element can be chosen in a group comprising a pin, a screw, a rivet or other similar or comparable elements.

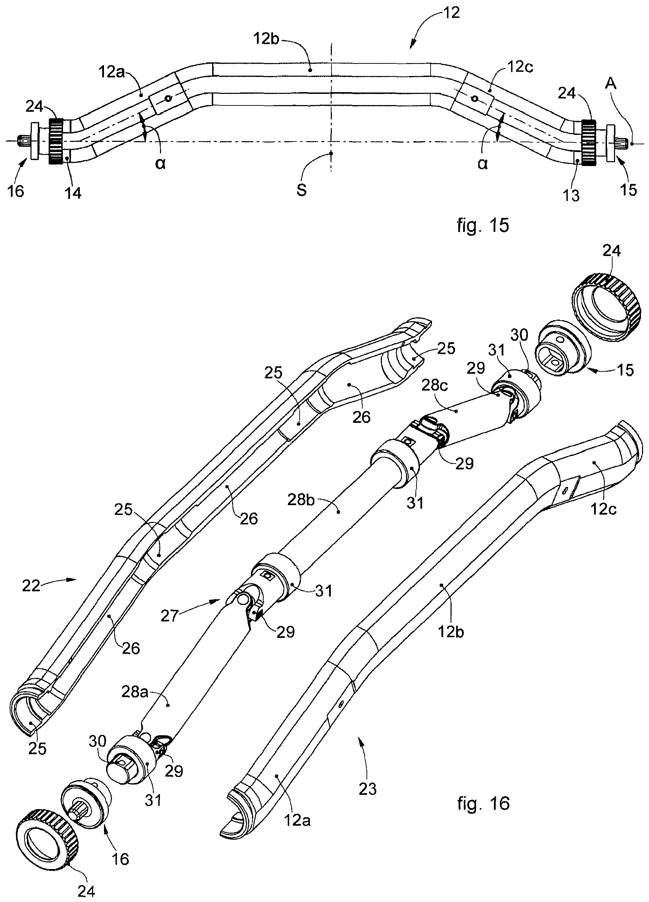

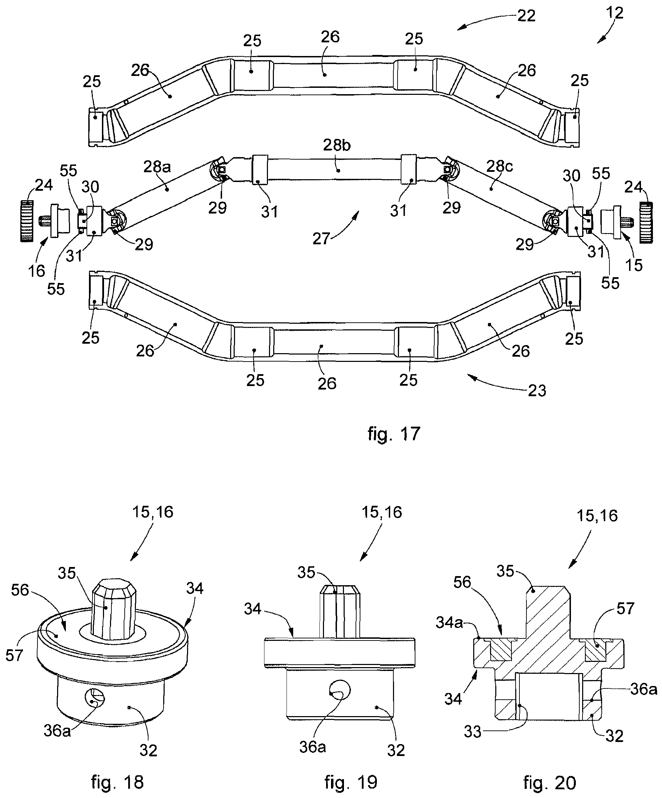

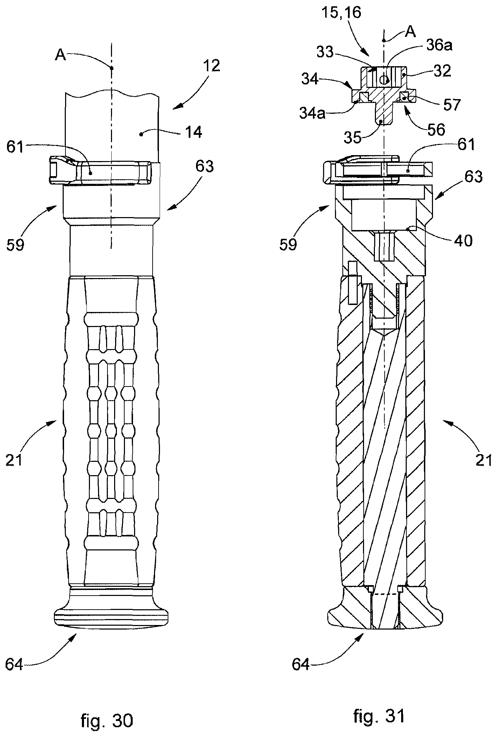

In accordance with some embodiments, shown in figs. 15-31 the base 32 is provided, on a lateral surface, with connection holes 36a (figs. 18-19) which during use are aligned and house a respective interference element 55 (fig. 17) which is associated with the connection interface 30 in order to allow to make at least temporarily respectively the attachment portions 15, 16 integral with the transmission unit 27.

The plate 34 and the coupling head 35 are configured to cooperate with one of the acetabular milling cutters 17 as above, with the positioning element 20, with a connection adapter possibly fastened to a respective acetabular milling cutter 17, with another connection adapter 19 fastened to a motorized drive member, or directly to the transmission element 21.

For this purpose, the plate 34 is provided with a support surface 34a which, during use, is coordinated with and facing, on each occasion, striker surfaces 37, 39, 40 respectively of the acetabular milling cutter 17, of the connection adapters 19 and of the transmission element 21. In particular, the support surface 34a and the striker surfaces 37, 39, 40 can be coupled with more or less wide play, figs. 1-14, or they can be located in contact, figs. 15-31.

The coupling head 35 develops from the plate 34 in the direction of the operating axis A, on the opposite side to that of the base 32.

To promote the engagement of the coupling head 35 with the acetabular milling cutter 17, with the positioning element 20, with one of the connection adapters possibly fastened to a respective acetabular milling cutter 17, with one of the other connection adapters 19 fastened to the motorized drive member, or directly with the transmission element 21, the coupling head 35 can have a prismatic shape.

Especially in the case where the multifunction device is used as a milling device 10a, the prismatic shape of the coupling head 35 allows to improve the transmission of the rotary motion both on the drive member side, and also on the acetabular milling cutter 17 side.

In particular, the coupling head 35 can have a polygonal-shaped section, in particular, but not limited to, quadrangular (figs. 1-14), or hexagonal (figs. 15-31), to allow a selectively releasable connection with the acetabular milling cutter 17, with the positioning element 20, with one of the connection adapters possibly fastened to a respective acetabular milling cutter 17, with one of the other connection adapters 19 fastened to the motorized drive member, or directly with the transmission element 21.

The hexagonal shape of the coupling head 35 allows, once the coupling with the acetabular milling cutter 17 has been made, to distribute the connection force in a more homogeneous manner between the coupling head 35 and an attachment part 45 of the acetabular milling cutter 17 as above. With the same transmitted force, the hexagonal shape, also, allows to reduce the sizes of the aperture 46 of the acetabular milling cutter 17 that houses the coupling head 35 as above.

In accordance with some embodiments, the coupling head 35 has clamping means 56 configured for a stable connection with the acetabular milling cutter 17, with the positioning element 20, with one of the connection adapters possibly fastened to a respective acetabular milling cutter 17, with one of the other connection adapters 19 fastened to the motorized drive member, or directly with the transmission element 21.

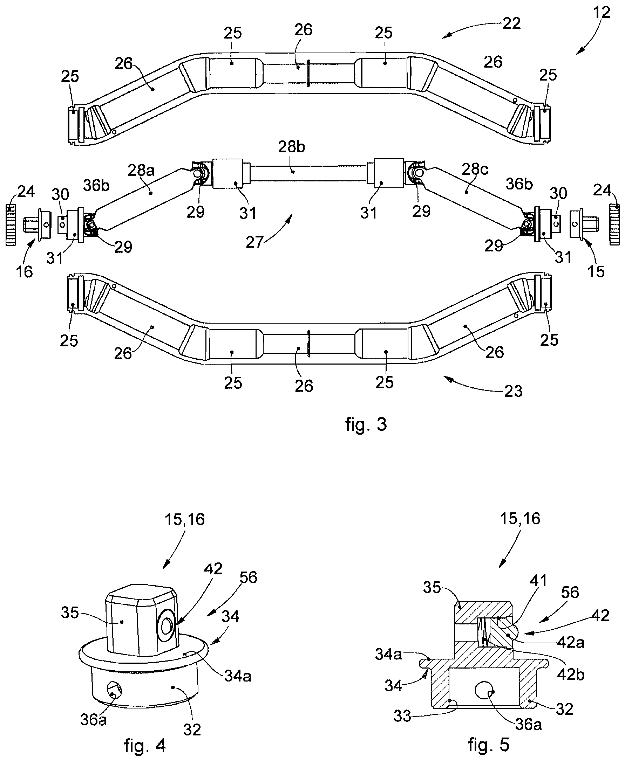

In accordance with some embodiments, shown in figs. 1-14, the clamping means 56 comprise at least one presser device 42 (see for example figs. 4-5, 8-9, 13-14) configured to generate an interference with the translation in the direction of the operating axis A in order to ensure the clamping of the acetabular milling cutter 17, of the positioning element 20, of the connection adapters 19, or of the transmission element 21. In this case, the coupling head 35 has internally a housing compartment 41, open toward the outside, to house the presser device 42 as above. For this purpose, at least one portion of the presser device 42 has to exit the profile of the coupling head 35 in a direction orthogonal to the operating axis A.

The housing compartment 41 is advantageously through so that possible organic residues, or possible condensation can easily be discharged during/after the washing and sterilization process.

In accordance with possible solutions, the presser device 42 can be chosen in a group comprising a spring presser, a ball presser, a spring and ball presser.

In the embodiment described here, the presser device 42 is of the spring and ball type and comprises an interference element 42a and an elastic element 42b that generates an elastic thrust on the interference element 42a in a direction orthogonal to the operating axis A.

In accordance with some embodiments, shown in figs. 15-31, the clamping means 56 comprise at least one magnetic element 57 (see for example figs. 18, 20, 23-24, 29, 31), for example a permanent magnet, such as an annular element, or ring, of magnetic material. The at least one magnetic element 57 is advantageously configured to selectively go on each occasion in abutment with striker surfaces 37, 39, 40 respectively of the acetabular milling cutter 17, of the connection adapters 19 and of the transmission element 21.

The magnetic element 57 can be inserted in a specific seating made in the plate 34 and can define part of the support surface 34a.

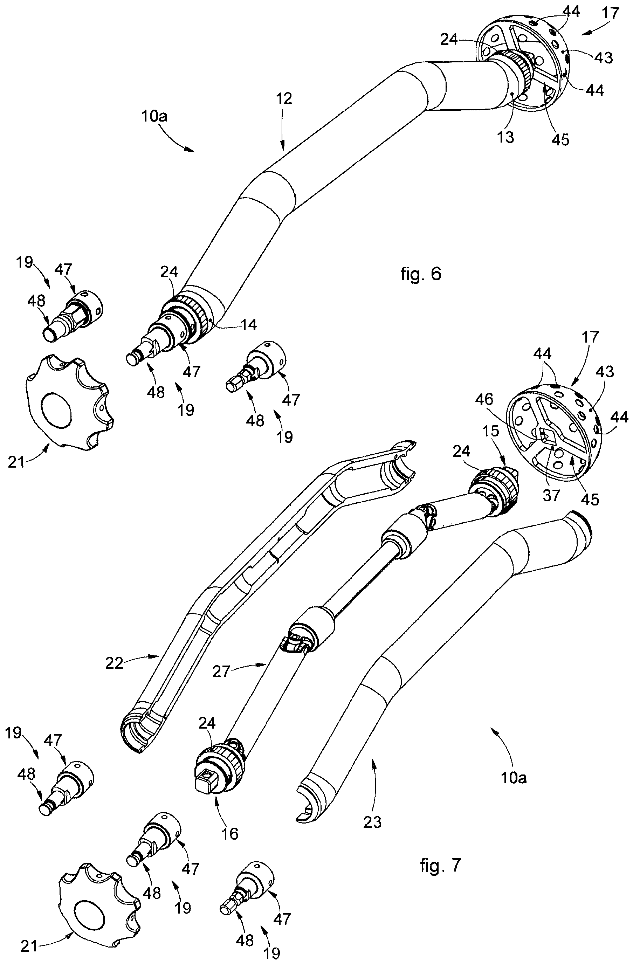

Some embodiments, shown in figs. 6-9 and figs. 21-24, concern a milling device 10a which comprises the handling body 12, one or more acetabular milling cutters 17, and possibly one or more connection adapters able to be fastened on one side directly to the distal attachment portion 15 and on the other side to a respective one of said acetabular milling cutters 17.

In accordance with possible solutions, the acetabular milling cutter 17 can comprise a support part 43 that has a substantially hemispherical shape internally hollow and configured to support a plurality of cutting edges 44 distributed on it, in a desired manner.

The acetabular milling cutter 17 can, also, comprise an attachment part 45 stably fixed to the base of the support part 43 and provided with the contact surface 37 and an aperture 46 configured to cooperate respectively with the plate 34 and with the coupling head 35 of the distal attachment portion 15 or of the proximal attachment portion 16.

Advantageously, the shape of the aperture 46 is mating with the shape of the coupling head 35 so that no connection adapters are needed between them.

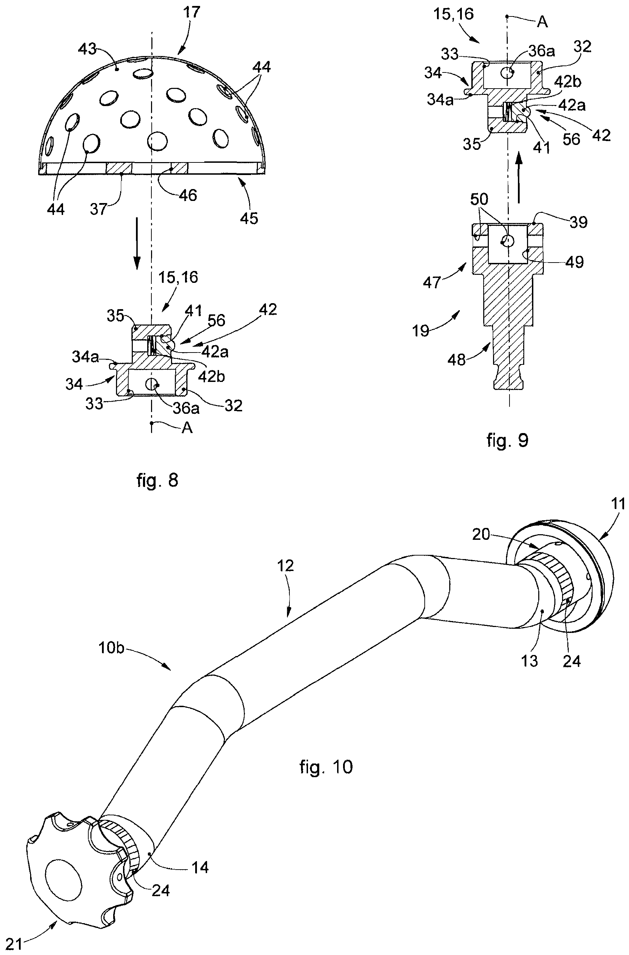

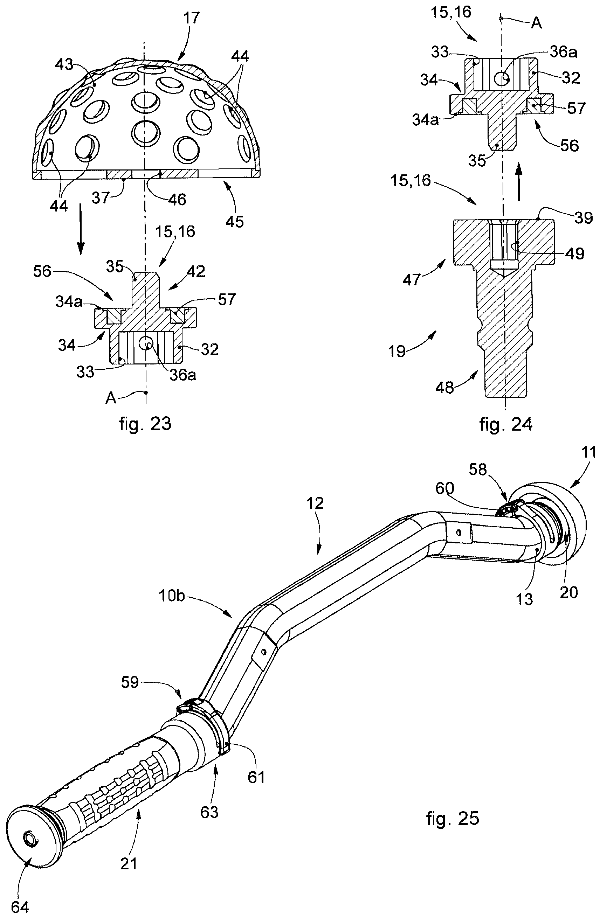

In accordance with the embodiment, shown in fig. 8 and in fig. 23, the acetabular milling cutter 17 is configured to couple directly with the distal attachment portion 15 or with the proximal attachment portion 16 with respect to the operating axis A.

In accordance with the embodiment, shown in figs. 6-9, the coupling head 35 once inserted into the aperture 46 in the direction of the operating axis A, will have penetrated therein by a height such that the presser device 42 is above the aperture 46 and below the support part 43 so as to axially clamp by interference the distal attachment portion 15 or the proximal attachment portion 16 with the acetabular milling cutter 17. At the same time the surface 37 of the attachment wall 45 can be resting on the support surface 34a of the plate 34 in order to increase the overall coupling surface and, therefore, improve the stability of the connection.

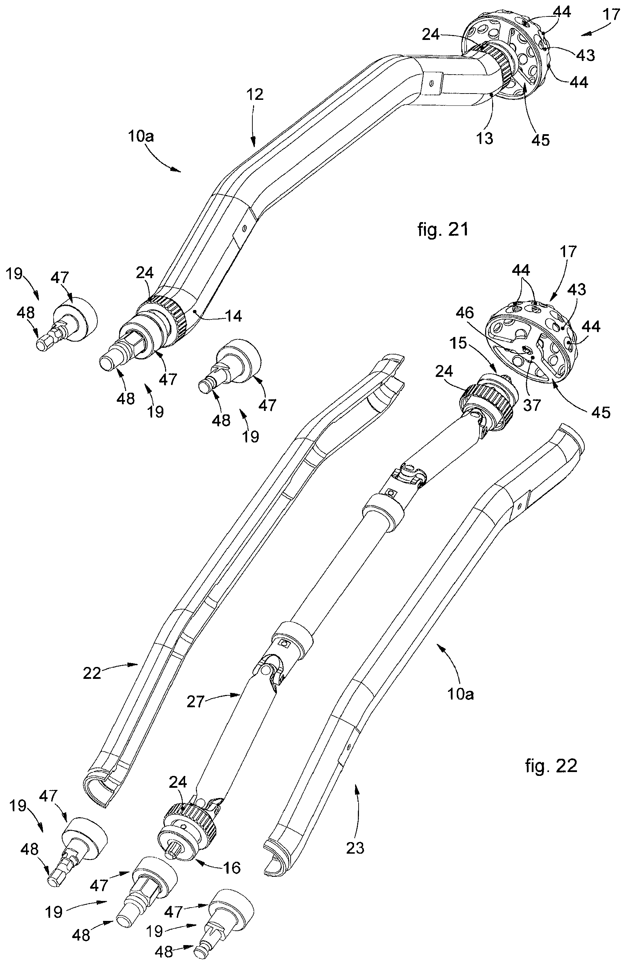

In accordance with the embodiment, shown in figs. 21-24, the coupling head 35 once inserted into the aperture 46 in the direction of the operating axis A, will have penetrated therein by height such that the support surface 34a part of which has the magnetic element 57 goes into contact with the surface 37 of the attachment wall 45 suitably made of a metal material that has magnetic properties which allow an attraction suitable to make a stable connection.

The milling device 10a, also, comprises one or more further connection adapters 19 able to be fastened as a replacement of the transmission element 21, in the milling configuration, on one side directly to a respective attachment portion 16, 15 and on the other side to a motorized drive member.

In accordance with the embodiment of fig. 7 and figs. 21-22 three connection adapters 19 are shown each of which is configured to operatively connect a different motorized drive member, not shown, which will have a mating coupling compartment, with one of said distal attachment portions 15 or proximal attachment portion 16.

Each connection adapter 19 comprises a universal attachment part 47, able to be fastened to the proximal attachment portion 16 or to the distal attachment portion 15, and a specialized attachment part 48, able to be fastened to the specific motorized drive member.

The universal attachment part 47 is provided with a connection cavity 49 that has a shape mating with that of the coupling head 35.

In accordance with the embodiment, shown in fig. 9, the universal attachment part 47 has at least one interference channel 50 passing from the connection cavity 49 toward the outside and suitable to at least partly house the presser device 42.

The coupling head 35 is inserted in the connection cavity 49 so that the presser device 42 is partly inserted in the interference channel 50 as above.

Advantageously, the universal attachment part 47 is provided with one or more interference channels 50 orthogonal to the operating axis A and angled with respect to each other so that the coupling head 35 is positioned so that the presser device 42 is aligned with any one of the angled directions of the interference channels 50.

Advantageously, the interference channels 50 are through toward the outside so that possible organic residues, or possible condensation can easily be discharged during/after the washing and sterilization process.

In accordance with the embodiment, shown in fig. 24, the connection between the coupling head 35 and the connection adapter 19 occurs between the support surface 34a part of which has the magnetic element 57 and the striker surface 39. Also in this case, the particular hexagonal shape of the coupling head 35 allows a more effective transmission of the torque.

In some cases, it is necessary to drive the milling device 10a by means of the transmission element 21. The transmission element 21 is configured to be connected directly to the distal attachment portion 15 or to the proximal attachment portion 16 and is typically driven by the surgeon in the final step of the process of making the acetabular seating.

Embodiments in which the multifunction device is converted into positioning device 10b are described using the figs. 10-14 and the figs. 25-31 and comprise the handling body 12 as above, the positioning element 20 able to be fastened on one side directly to the distal attachment portion 15 and on the other side to an acetabular cup 11 of a hip prosthesis.

The positioning element 20 comprises a connection body 51 provided with a connection seating 53 for housing the distal attachment portion 15 or the proximal attachment portion 16, and a holding element 52 for the connection to the acetabular cup 11.

The connection body 51 advantageously has a flared shape in the direction of the holding element 52 so as not to interfere at other points with the acetabular cup 11.

In accordance with the embodiment, shown in figs. 25-31, the positioning element 20 and the transmission element 21 are provided with respective releasable clamping devices 58, 59 which allow respectively to make the positioning element 20 integral with the handling body 12 and to clamp the rotation of the transmission element 21 which determines the motion of the transmission unit 27.

In this way, once the correct position of the acetabular cup 11 with respect to suitable surgical references has been determined, this position remains unchanged even in case of accidental movements of the surgeon during the operating technique, or during the strikes inflicted by the striker member to transmit the impact force.

The releasable clamping devices 58, 59 each comprise a respective tightening lever 60, 61 which allows to selectively make integral respectively the positioning element 20 and the transmission element 21 with the handling body 12.

The tightening lever 60 of the releasable clamping device 58 of the positioning element 20 has an open position, suitable to allow the engagement of the positioning element 20 on an attachment portion 15, 16 and its correct angular position with respect to the operating axis A, and a closing position, suitable to make the positioning element 20 integral with the handling body 12.

The closing position is necessary to guarantee that, during the impact action of the striker member on the transmission element 21 for the fixing in position of the acetabular cup 11 in the acetabular seating, made previously or already present, the strike inflicted transmits the force required for the fixing as above. In fact, otherwise, the strike inflicted by the striker member could be ineffective and transmit the impact force onto the surgeon's arm.

The correct angular position with respect to the operating axis A is the one that guarantees the minimum bulk of the tightening lever 60 during the surgical technique and can be defined by aligning suitable references on the handling body 12 and on the positioning element 20.

In addition, in operating techniques of revision of prosthetic implants of the hip, the opening position allows a rotation of the acetabular cup 11, previously fixed to the positioning element 20, to allow a correct alignment thereof for the insertion of fixing screws.

The tightening lever 61 of the releasable clamping device 59 of the transmission element 21 has an open position, suitable to allow the engagement of the transmission element 21 on an attachment portion 15, 16 and its rotation with respect to the operating axis A in order to activate the transmission unit 27 and transmit the rotary motion from the distal end 13 to the proximal end 14, and a closing position, suitable to clamp the transmission element 21 to the handling body 12 preventing the activation of the transmission unit 27.

The tightening levers 60, 61 are configured to at least partly wind the handling body 12 in correspondence with the distal end 13 and the proximal end 14 or vice versa. In particular, the tightening action of the tightening levers 60, 61 as above acts on the respective portion of the handling body 12 close to the ring nuts 24 on the opposite side with respect to the attachment portions 15, 16.

In accordance with the embodiment, shown in figs. 10-14, the holding element 52 is fixed.

In accordance with the embodiment, shown in figs. 25-31, the holding element 52 is mobile in rotation, about the operating axis A, in a housing cavity 62 of the positioning element 20.

The housing cavity 62 is open on one side toward the connection seating 53, so as to allow the holding element 52 to connect with the attachment portion 15, 16 or with the attachment portion 16, 15, and on the opposite side toward the outside so as to allow the connection of the holding element 52 with the acetabular cup 11.

The holding element 52 has a threaded tip for the anchoring to the acetabular cup 11.

In particular, when the tightening lever 60 of the releasable clamping device 58 of the positioning element 20 is in the closing position and the tightening lever 61 of the releasable clamping device 59 of the transmission element 21 is in the open position, it is possible, by rotating the latter, to firmly screw the acetabular cup 11 to the positioning element 20.

Furthermore, the screwing action allows to compact the holding element 52 and the acetabular cup 11 toward the attachment portion 15, 16 creating a very stable tightening. In accordance with the embodiment, shown in figs. 25-31, the transmission element 21 has the shape of a handle intended for the grip to rotate and position the acetabular cup 11 as described above and intended to be struck by a striker member to fix the acetabular cup 11 in its seating.

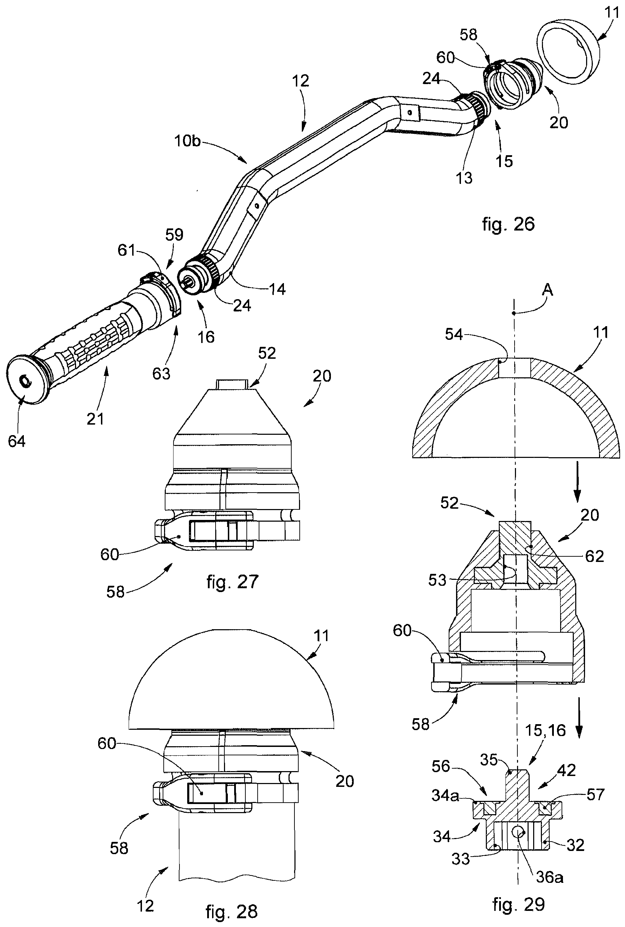

In particular, fig. 28-29, the transmission element 21 comprises a coupling end 63 provided with the releasable clamping device 59 and an opposite abutment end 64 suitable to be struck by a striker member, for example by a surgical hammer.

The coupling end 63 is, also, provided with a coupling seating to receive one of the attachment portions 15, 16.

The acetabular cup 11 has a substantially hemispherical hollow shape and is provided, on its top, with a holding hole 54 in which the holding element 52 of the positioning element 20 is temporarily inserted, by interference (fig. 10-14) or by screwing (figs. 25-31).

In accordance with one aspect of the present invention, the transmission element 21 and the positioning element 20 are resting only on the corresponding ring nuts 24 of the handling body 12, in the direction of the operating axis A, so that the strike of the striker member on the transmission element 21 is transmitted through the handling body 12 onto the positioning element 20 without affecting the transmission unit 27.

In this way, once the strike is generated on the transmission element 21, the acetabular cup 11 disengages from the holding element 52 of the positioning element 20 and remains in position in the acetabular seating made with the milling device 10a waiting to be permanently fixed therein by means of for example, screws or surgical cement.

In the event the acetabular cup 11 is of the helicoidal type, the positioning device 10b is, also, suitable to screw it into the acetabular seating made with the milling device 10a.

In accordance with one aspect of the present invention, at least the handling body 12 and the acetabular milling cutter 17 are made of biocompatible and hypoallergenic metal material.

In preferred embodiments, at least the handling body 12 and the acetabular milling cutter 17 are made of titanium which ensures high biocompatibility with the human body preventing problems of postoperative rejection; it is in fact biocompatible and hypoallergenic.

Advantageously, the connection adapters, the additional connection adapters 19, the transmission element 21, and the positioning element 20 can also be made of biocompatible and hypoallergenic metal material such as, for example, titanium.

In other embodiments, at least the handling body 12 and the acetabular milling cutter 17 are made of steel.

Embodiments of the present invention concern a method to use the multifunction device for prosthetic surgery described above.

The method to use the multifunction device provides the conversion from a milling configuration, to be used as a milling device 10a for prosthetic hip surgery, to a positioning configuration, to be used as a positioning device 10b to position an acetabular cup 11 of a hip prosthesis; the method as above comprises:
- making available a single tubular oblong handling body 12 which develops along the operating axis A, provided with the distal end 13 and the proximal end 14 opposite each other, inside the handling body 12 there is the transmission unit 27 of the rotary motion from the distal end 13 to the proximal end 14, the transmission unit 27 ending in the respective attachment portions 15, 16, respectively distal and proximal, which are interchangeable with each other;
- when the device is converted into the milling configuration as above, releasably connecting the specific acetabular milling cutter 17 to one of the attachment portions 15, 16 in the milling configuration as above;
- when the device is converted into the positioning configuration as above, releasably fastening the positioning element 20 on one side directly to one of the attachment portions 15, 16 and on the other side to the acetabular cup 11 of a hip prosthesis;
- when the device is converted into the positioning configuration as above, releasably fastening the transmission element 21 to one of the attachment portions 16, 15 opposite the attachment portion 15, 16 to which the acetabular milling cutter 17 or the positioning element 20 is fastened, the transmission element 21, in the milling configuration as above or respectively in the positioning configuration as above, transmitting, by means of the transmission unit 27, a rotation from one of the attachment portions 16, 15 to the other one of the attachment portions 15, 16, respectively associated with the specific acetabular milling cutter 17 or with the acetabular cup 11 associated with the positioning element 20, or again the positioning element 20, in the positioning configuration as above, is struck by a striker member to transmit an impact force, through the handling body 12 and the positioning element 20, to forcefully position the acetabular cup 11 associated with the positioning element 20.

It is clear that modifications and/or additions of parts and/or steps may be made to the multifunction device for prosthetic surgery and to the corresponding method of use as described heretofore, without departing from the field of the present invention as defined by the claims.

It is also clear that, although the present invention has been described with reference to some specific examples, a person of skill in the art shall certainly be able to achieve many other equivalent forms of multifunction device for prosthetic surgery and corresponding method of use, having the characteristics as set forth in the claims and hence all coming within the field of protection defined thereby.

In the following claims, the references in brackets have the sole purpose to facilitate reading and they must not be considered as restrictive factors with regard to the field of protection claimed in the specific claims.

## Claims

1. Multifunction device for prosthetic surgery able to be converted from a milling configuration, to be used as a milling device (10a) for hip prosthetic surgery, to a positioning configuration, to be used as a positioning device (10b) for positioning an acetabular cup (11) of a hip prosthesis, said multifunction device comprising:
- a single tubular oblong handling body (12), which develops along an operating axis (A), provided with a distal end (13) and a proximal end (14) opposite each other, inside the handling body (12) there is a transmission unit (27) of the rotary motion from the distal end (13) to the proximal end (14), the transmission unit (27) ending in respective attachment portions (15, 16), respectively distal and proximal, which are interchangeable with each other;
- one or more acetabular milling cutters (17) able to be releasably connected to one of said attachment portions (15, 16) in said milling configuration;
- a positioning element (20) able to be releasably fastened on one side directly to one of said attachment portions (15, 16) and on the other side to an acetabular cup (11) of a hip prosthesis, in said positioning configuration;
- a transmission element (21) able to be releasably fastened to one of said attachment portions (16, 15) opposite the attachment portion (15, 16) to which said acetabular milling cutter (17) or said positioning element (20) is fastened, the transmission element (21) being configured so that, in said milling configuration or respectively said positioning configuration, it can be used to transmit, by means of said transmission unit (27), a rotation from one of said attachment portions (16, 15) to the other of said attachment portions (15, 16), respectively associated with a specific acetabular milling cutter (17) or with said acetabular cup (11) associated with said positioning element (20), or again said transmission element (21), in said positioning configuration, is able to be struck by a striker member in order to transmit an impact force, through said handling body (12) and said positioning element (20), to forcefully position said acetabular cup (11) associated with said positioning element (20).

2. Device as in claim 1, **characterized in that** said attachment portions (15, 16) are, in particular, the same as each other and interchangeable, each comprising a base (32), a coupling head (35) and a plate (34) located intermediate.

3. Device as in claim 2, **characterized in that** said coupling head (35) has a hexagonal-shaped section.

4. Device as in claim 2 or 3, **characterized in that** said coupling head (35) is provided with clamping means (56) configured to make a stable connection selectively with the acetabular milling cutter (17), with the positioning element (20), with one of the connection adapters possibly fastened to a respective acetabular milling cutter (17), with one of the additional connection adapters (19) fastened to the motorized drive member, or directly with the transmission element (21).

5. Device as in claim 4, **characterized in that** said clamping means (56) comprise at least one magnetic element (57).

6. Device as in any claim hereinbefore, **characterized in that** the positioning element (20) and the transmission element (21) are provided with respective clamping devices (58, 59) which allow respectively to make the positioning element (20) integral with the handling body (12) and to clamp the rotation of the transmission element (21) which determines the motion of the transmission unit (27).

7. Device as in claim 6, **characterized in that** said clamping devices (58, 59) each comprise a respective tightening lever (60, 61) configured to selectively make integral respectively the positioning element (20) and the transmission element (21) with the handling body (12).

8. Device as in claim 7, **characterized in that** said tightening lever (60) has an open position, suitable to allow the engagement of the positioning element (20) on an attachment portion (15, 16) and to allow its correct angular position with respect to the operating axis (A), and a closing position, suitable to make the positioning element (20) integral with the handling body (12).

9. Device as in claim 7, **characterized in that** said tightening lever (61) has an open position, suitable to allow the engagement of the transmission element (21) on an attachment portion (15, 16) and its rotation with respect to the operating axis (A) to activate the transmission unit (27) and transmit the rotary motion from the distal end (13) to the proximal end (14), and a closing position, suitable to clamp the transmission element (21) to the handling body (12) preventing the activation of the transmission unit (27).

10. Device as in claim 5, **characterized in that** said acetabular milling cutter (17) comprises an attachment part (45) provided with a striker surface (37) and with an aperture (46) which are configured to cooperate respectively with said plate (34) and with said coupling head (35) of the respective attachment portion (15, 16).

11. Device as in any claim hereinbefore, **characterized in that** it comprises one or more further connection adapters (19) able to be fastened as a replacement of said transmission element (21), in said milling configuration, on one side directly to a respective attachment portion (16, 15) and on the other side to a motorized drive member.

12. Device as in claim 11, **characterized in that** each connection adapter (19) comprises a universal attachment part (47), able to be fastened to a respective attachment portion (16, 15), and a specialized attachment part (48), able to be fastened to the specific motorized drive member.

13. Device as in any claim hereinbefore, **characterized in that** said positioning element (20) comprises a connection body (51) provided with a connection seating (53) to house the respective attachment portion (15, 16), and a holding element (52) for the temporary connection to the acetabular cup (11).

14. Device as in claim 13, **characterized in that** said holding element (52) is mobile in rotation about the operating axis (A) in a housing cavity (62) of the positioning element (20).

15. Device as in any claim hereinbefore, **characterized in that** said handling body (12) is provided with ring nuts (24) present on both said distal end (13) and also said proximal end (14), coaxially with the respective attachment portions (15, 16), wherein, in said positioning configuration, said transmission element (21) and said positioning element (20) are able to be resting exclusively on said ring nuts (24), in the direction of the operating axis (A).

16. Device as in any claim hereinbefore, **characterized in that** at least the handling body (12) and the acetabular milling cutter (17) are made of biocompatible and hypoallergenic metal material, in particular titanium.

17. Device as in any claim hereinbefore, **characterized in that** said handling body (12) has an elongated conformation in the direction of said operating axis (A), passing through the attachment portions (15, 16), and comprises a first shell (22) and a mating second shell (23) able to be stably coupled to each other in a releasable manner in order to house, inside, said transmission unit (27).

18. Device as in any claim hereinbefore, **characterized in that** said handling body (12) has a symmetrical conformation with respect to a central axis (S) passing through the center of the handling body (12) and orthogonal to said operating axis (A).

19. Device as in any claim hereinbefore, **characterized in that** said handling body (12) has a symmetrical conformation with respect to a coupling plane with respect to which the first shell (22) and the second shell (23) are coupled and which passes through said operating axis (A).

20. Method to use a multifunction device for prosthetic surgery in a milling configuration, to be used as a milling device (10a) for hip prosthetic surgery, and in a positioning configuration, to be used as a positioning device (10b) for positioning an acetabular cup (11) of a hip prosthesis, said method comprising:
- making available a single tubular oblong handling body (12) which develops along an operating axis (A), provided with a distal end (13) and a proximal end (14) opposite each other, inside the handling body (12) there is a transmission unit (27) of the rotary motion from the distal end (13) to the proximal end (14), the transmission unit (27) ending in respective attachment portions (15, 16), respectively distal and proximal, which are interchangeable with each other;
- when the device is converted into said milling configuration, releasably connecting a specific acetabular milling cutter (17) to one of said attachment portions (15, 16);
- when the device is converted into said positioning configuration, releasably fastening a positioning element (20) on one side directly to one of said attachment portions (15, 16) and on the other side to an acetabular cup (11) of a hip prosthesis;
- releasably fastening a transmission element (21) to one of said attachment portions (16, 15) opposite the attachment portion (15, 16) to which said acetabular milling cutter (17) or said positioning element (20) is fastened, the transmission element (21), in said milling configuration or respectively said positioning configuration, transmitting, by means of said transmission unit (27), a rotation from one of said attachment portions (16, 15) to the other of said attachment portions (15, 16), respectively associated with a specific acetabular milling cutter (17) or with an acetabular cup (11), or again said positioning element (20), in said positioning configuration, is struck by a striker member in order to transmit an impact force, through said handling body (12) and said positioning element (20), to forcefully position an acetabular cup (11) associated with said positioning element (20).
